# EUROPEAN PATENT APPLICATION

(11) **EP 0 626 445 A2**
(43) Date of publication of application: **30.11.1994**
(21) Application number: 94112077.6
(22) Date of filing: 24.08.1989
(51) Int. Cl.: C11D 3/386

(54) **Detergent compositions**

(30) Priority: 24.08.1988 GB 8820061; 24.08.1988 GB 8820062; 19.01.1989 GB 8901182
(62) Divisional of application: 89308607.4
(71) Applicant: ALLIED COLLOIDS LIMITED, Bradford, West Yorkshire BD12 0JZ (GB)
(72) Inventor: Langley, John Graham, Leeds, West Yorkshire LS20 8NS (GB); Symes, Kenneth Charles, Keighley, West Yorkshire BD20 2UU (GB)
(74) Representative: Lawrence, Peter Robin Broughton

(57) **Abstract**

Enzyme for inclusion in liquid detergent concentrates is provided as a stable dispersion of small particles that also include a protective polymeric material which may be matrix polymer, a coacervate shell, either of which may be present together with a film of polymeric dispersion stabiliser, or a film of dispersion stabilizer alone.

## Description

It is well known to include enzymes in detergent compositions in order to improve their performance. The enzymes will be de-activated by exposure to other chemical components in the detergent, and possibly also upon exposure to moisture. It is therefore necessary to protect the enzyme, for instance by encapsulation within a polymer. Detergent powders therefore frequently contain powdered enzyme-polymer compositions.

A difficulty that exists with liquid detergents is that the liquid phase of the detergent concentrate is more likely to attack the encapsulating polymer and does, in any event, tend to provide a more rigorous chemical and physical environment from which the polymer has to protect the enzyme. It has proved difficult to provide the enzyme in a protected form and that is stably distributed within the liquid detergent. If the enzyme is not fully protected then the enzyme activity in the detergent, when it is used by the consumer, will be very low.

In GB 2,186,884 it is proposed to disperse the enzyme in a silicone oil and to disperse this dispersion in the liquid detergent. Although it is proposed that the dispersed particles can have a size as low as 2µm in practice they are always very much larger than this. This has to follow from the fact that the enzyme, that is within the dispersed particles, is stated to have a particle size that is preferably above 5µm, with the result that the dispersed particles containing the enzyme must inevitably be much larger. In practice therefore at least 90% of the particles of oil and enzyme will always have a particle size substantially above 5µm.

In JP-A-63-105098 it is proposed to microencapsulate enzyme in polyvinyl alcohol and to disperse the resultant microcapsules in a liquid or gel detergent. In practice any liquid detergents that are used would need to have a high viscosity because the microcapsules that are made are always relatively large, for instance 150 to 800µm in example 1 and so would inevitably settle out from a conventional, relatively low viscosity, liquid detergent concentrate. One way that is described for making the microcapsules involves dispersing an enzyme solution containing polyhydroxy compound and polyvinyl alcohol is dispersed in hydrophobic solvent as microdroplets and the water content is removed by heating or decrease in pressure. As mentioned, the particle size is large and this process is not exemplified.

The commercial reality is that there is no entirely satisfactory way of incorporating detergent enzymes into liquid detergent concentrates and, in particular, the enzymic activity of any such composition is rapidly lost during storage.

It would be desirable to be able to provide a liquid composition that can be incorporated into a liquid detergent concentrate, and it would be desirable to provide such concentrates wherein the enzymic activity can be maintained.

A liquid composition according to the invention comprises a substantially stable dispersion in a liquid phase of particles comprising a detergent enzyme and a protective polymeric material that is impermeable to liquid detergent concentrates but releases the enzyme when agitated in aqueous wash liquor and that comprises an outer polymeric shell and/or a polymeric matrix through which enzyme is distributed, and in this composition the particles have a size below 20µm and have been made by a process comprising (a) forming a dispersion of an aqueous liquid phase containing the enzyme in a water immiscible liquid in the presence of a dispersion stabiliser and azeotroping the dispersion, and/or (b) forming an outer polymeric shell by coacervation.

Part at least of the process for making the composition preferably comprises forming a dispersion of an aqueous phase containing the enzyme in a water immiscible liquid in the presence of the dispersion stabiliser and azeotroping the dispersion. The azeotroped produced may serve as the liquid composition, in which event the liquid phase of the composition is the water immiscible liquid in which the dispersion was formed. The particles in this dispersion (and in the final product) are preferably below 10µm, most preferably below 3µm. This assists the formation of a stable dispersion.

The dispersion stabiliser that is used in this process is preferably an amphipathic polymeric stabiliser, that is to say a polymeric stabiliser having hydrophobic and hydrophilic components as a result of having been made from hydrophobic and hydrophilic monomers. Accordingly, the particles of the dispersion each have a surrounding hydrophobic film derived from the hydrophobic units in the stabiliser and this hydrophobic film can serve as part or all of the protective polymeric material. The formation of the initial dispersion, that is subjected to azeotroping, is generally facilitated by the incorporation of a water-in-oil emulsifier, and the hydrophobic groups of this may also contribute to the formation of a protective hydrophobic film around each particle.

Generally, however, it is necessary to include additional polymeric material in order to provide adequate protection. This additional material can be a polymeric matrix for each particle, with the enzyme being distributed through the matrix. The matrix may be of uniform composition throughout, but preferably it is chemically modified during or after the formation of the matrix (i.e., during or after the azeotroping) so as to render it less permeable to liquid detergent concentrate. Thus the polymer may be introduced in soluble form and may then be insolubilised, particularly at the outer surface layer of the matrix.

Instead, and usually in addition to, having a matrix polymer, it is also possible to provide an outer polymeric shell. This can be formed by any suitable microencapsulation technique but, in the invention, it is particularly preferred that it is formed by coacervation. Coacervation is usually conducted in an aqueous phase and so the liquid phase of the composition of the invention can be this coacervating liquid phase, provided the polymeric shell is sufficiently impermeable to it during storage.

The liquid compositions of the invention also include compositions wherein the liquid phase is the continuous phase of a liquid detergent concentrate. This may be formulated in conventional manner from appropriate blends of surfactants, builders and other conventional additives for liquid detergents.

The liquid detergent is normally a relatively low viscosity liquid that has a low water content and a high content of surfactant and/or electrolyte. Many polymeric materials, particularly if they are addition polymers made from a monomer or monomer blend containing ionic monomers, are much less soluble and permeable when exposed to high electrolyte or surfactant concentrations than when exposed to dilute aqueous solutions. Because of this, and because of the small particle size of the particles, it is therefore possible to provide the enzyme in a protective polymer that is substantially impermeable to the other components of the liquid detergent but which will dissolve when the water content is increased greatly, namely when the concentrate is diluted into the wash liquor for use. Although the dilution effect alone may be sufficient to release the enzyme from within the particles, reliance may also be placed on the temperature of the wash liquor, since this is generally above 30°C and usually above 40°C and so is above the normal storage temperature of the composition. In particular, reliance may be placed on agitation, for instance of the sort to which aqueous wash liquors are normally subjected, since this can promote rupture of the polymeric matrix or, especially, of any outer protective polymeric shell.

The enzyme can be any enzyme that is useful in detergents. It is preferably a protease, especially an alkaline protease, but it may for instance be an amylase or a lipase. It may be introduced initially in powder form but is generally introduced as a solution (or a dispersion containing cellular material with which it was initially produced). This solution may be formed from a dried enzyme product or it may be a fermentation liquor, for instance the fermentation broth in which the enzyme was produced initially or a concentrate obtained from that. Processes in which the enzyme is provided initially as a fermentation broth are described in EP-A-356240.

The preferred products of the invention are made by a process that involves reverse phase azeotroping, and for this purpose an aqueous phase containing the enzyme is dispersed in a water immiscible liquid in the presence of the dispersion stabiliser. The aqueous phase should be stable both against phase separation and against loss of activity of the enzyme. For instance it can be desirable to include a polyhydroxy compound, especially sucrose or other sugar or a glycol or other low molecular weight polyhydroxy compound, e.g., propylene glycol. The dispersion of the aqueous phase into the water immiscible liquid is generally accompanied by shear and is conducted in the prescence of a water-in-oil emusifier so as to promote the formation of small particles generally having a size below 10µm, most usually below 3µm. Suitable surfactants, water immiscible liquids and polymeric stabilisers, and suitable azeotroping conditions, are described in EP 0128661 and 0126528 and, in particular, suitable stabilisers are described in GB 2,002,400 with particularly preferred stabilisers being described in GB 2,001,083 and GB 1,482,515.

The immiscible liquid is non-aqueous and must include liquid that will form an azeotrope with water. Often the water immiscible liquid is a blend of a relatively high boiling liquid that remains in the dispersion and a low boiling liquid that is azeotroped from the dispersion. The temperature at which azeotroping occurs is generally below 100°C and is controlled by the choice of liquid and, especially, the pressure at which the distillation is conducted. Generally the distillation is conducted under reduced pressure and when the active ingredient is temperature sensitive (e.g., an enzyme) the reduced pressure is preferably such that the azeotroping occurs at a maximum temperature of not more than 80°C, often below 70°C and most preferably below 50°C. For instance by applying a relatively high vacuum it is possible to azeotrope at very low temperatures, for instance as low as 30°C. Sodium sulphate or other salt may be added to lower the azeotroping temperature.

The polymer should be film forming at the distillation temperature, and usually is film forming at 20°C or lower. The azeotroping should be conducted to render the particles substantially dry so that they are not desensitised by the presence of water in them. In practice this means that the water content is generally below 25%, and preferably below 10%, by weight of the particles and most preferably is at or, especially, below the moisture content that would prevail if the particles were exposed to the atmosphere.

The aqueous phase containing the enzyme preferably contains a polymer so as to form a polymeric matrix through which the enzyme is distributed.

The polymer can be a natural or modified polymer such as a starch or a cellulose (e.g., carboxy methyl cellulose) or gum. Preferably it is a synthetic polymer formed from an ethylenically unsaturated water soluble monomer or monomer blend, which may be non-ionic or ionic.

Suitable anionic monomers are ethylenically unsaturated carboxylic or sulphonic monomers, most preferably monomers such as (meth) acrylic acid, crotonic acid, itaconic acid, maleic acid, (meth) allyl sulphonic acid, vinyl sulphonic acid and 2-acrylamido-2-methyl propane sulphonic acid. Acrylic or methacrylic acid is preferred.

Suitable cationic monomers are dialkylaminoalkyl (meth) -acrylamides and, preferably, -acrylates, usually as acid addition or quaternary ammonium salts. Particularly preferred are monomers such as diethylaminoethyl (meth) acrylate.

Suitable non-ionic monomers of this type are (meth) acrylamide and hydroxy-lower alkyl (meth) acrylates. The anionic and cationic monomers may be either in the free acid or free base form when they are sufficiently soluble in this form (for instance acrylic acid) but more usually in the form of an alkali metal or ammonium salt of anionic monomers or an acid addition or quaternary ammonium salt of cationic monomers.

The polymer may be polyvinyl pyrollidone, polyvinyl alcohol or ethylene (meth) acrylic acid copolymer.

The preferred polymer is usually based on 0-50% acrylamide and 50-100% acrylic acid or soluble salt thereof.

The soluble polymer may have been made by any conventional polymerisation technique, such as reverse phase suspension polymerisation, solution polymerisation, reverse phase bead polymerisation or gel polymerisation. Alternatively, the polymer may be a copolymer of soluble and insoluble monomers (e.g., methacrylic acid and ethyl acrylate) and may have been made by oil-in-water emulsion polymerisation followed by addition of sodium hydroxide or other alkali to convert it to a soluble form.

Instead of introducing the polymer in a soluble form, the polymer can be a polymer that is insoluble in water but is soluble in alkali and which is introduced as an oil-in-water emulsion that has been made by emulsion polymerisation of ethylenically unsaturated monomer or monomer blend that is insoluble in the water phase of the polymerisation mixture. The monomers are generally a blend of anionic solubilising monomers (typically selected from the anionic monomers discussed above) and ethylenically unsaturated non-ionic monomers, the overall blend being insoluble at the pH of the emulsion. Thus the emulsion polymerisation may be conducted at a pH below 7 but when the polymer is subsequently exposed to more alkaline conditions the polymer becomes soluble (or highly swellable). Suitable non-ionic water insoluble monomers include alkyl (meth) acrylates, styrene, acrylonitrile, vinyl chloride, vinyl acetate or vinyl butyl ether. Ethyl acrylate is preferred, with the polymer preferably being formed from 10 to 70% methacrylic acid or other anionic monomer, 10 to 70% ethyl acrylate or other insoluble monomer and 0 to 70% acrylamide or other soluble non-ionic monomer.

The use of an emulsion polymer of this type is of particular value when it is desired for the polymeric matrix to permit substantially no release of the biological material in one environment (for instance neutral or acidic) and rapid release in an alkaline environment.

Controlled release of enzyme can also be obtained when the polymer is introduced initially as a salt with a volatile amine (for instance ammonia) of a polymer derived from ethylenically unsaturated carboxylic acid monomer such as (meth) acrylic acid. The salt is soluble in water but the ammonia or other volatile amine evaporates during the azeotroping to render the polymer less hydrophilic. Accordingly at least the outer shell of the particles, and possibly substantially the entire polymeric matrix, will be less hydrophilic and water soluble than when the carboxylic groups are in alkali or amine salt form. The particle therefore has relatively low permeability to ambient moisture but, upon exposure to a slightly alkaline aqueous solution (for instance as typically prevails in a wash liquid) the polymer will be sufficiently solubilised to permit rapid release of the trapped enzyme or other biological material. For this purpose the polymer is preferably based on 0 to 50% acrylamide and 50 to 100% acrylic acid or, preferably, methacrylic acid. Products of these types are described in more detail in EP-A-361677.

The matrix polymer preferably is a polymer that is useful as a component in a detergent, for instance as a detergent builder or detergent anti-redeposition aid. Suitable polymers include carboxy methyl cellulose and anionic synthetic polymers, for instance polymers of molecular weight 4,000 to 300,000 and formed from water soluble ethylenically unsaturated carboxylic or sulphonic monomer, optionally with water soluble non-ionic monomer. Preferably the polymer is of sodium polyacrylate but copolymers with acrylamide and homopolymers or copolymers with, for instance, allyl sulphonate or 2-acrylamido methyl propane sulphonate may be used. Copolymers of maleic anhydride with, for instance, acrylic acid are also suitable.

It is preferred that the amount of polymer should be at least 0.5 times the amount of active ingredient (on a dry weight basis). Preferably there is an excess of polymer, for instance at least two times the amount of active ingredient and preferably there is a large excess, for instance at least seven times. The use of this seven-fold excess (or more) of polymer means that the biological material is protected very effectively from the environment and even if the particles are damaged the amount of active ingredient that is liable to be exposed to, or escape into, the environment is very low. Generally the amount of polymer is at least 10 times and usually at least 15 times the amount of active ingredient (on a dry weight basis). It is usually unnecesary for it to be more than 50 times, and amounts in the range 15 to 30 times are often suitable although amounts up to 100 times or more can be used.

Small amounts of filler and other additives can be included in the matrix. Generally the polymeric material constitutes at least 50%, preferably at least 75% and most preferably at least 90% by weight of the solid composition formed of a matrix, active ingredient and any inert material distributed through the matrix.

It is generally preferred that the aqueous phase that is to be dispersed and then azeotroped should be formed from the enzyme and preformed polymer. However if desired it can be made by polymerising the appropriate monomer or monomer blend in the presence of the enzyme so as to form the polymer in the presence of the enzyme. Alternatively, reactive polymer can be used, e.g., as in EP-A-032831 and can be reacted in the matrix or shell.

The product resulting from the azeotroping is a stable dispersion in the water immiscible liquid of substantially dry particles of which at least 90% by weight of which are preferably below 3µm, often below 2µm and frequently even below 1µm.

The polymeric particles will, in practice, generally have an absorbed layer of hydrophobic material due to emulsifier and/or polymeric stabiliser.

The matrix polymer is preferably chemically modified at least at its outer surface either during or after the azeotroping. For instance a volatile amine salt can be converted to a less volatile free acid compound or the polymer can be subjected to external chemical reaction. For instance if the polymer is formed of chitosan it can be acetylated, or otherwise esterified, so as to insolubilise it. Various other insolubilisation reactions can be conducted on water soluble polymers in known manner so as to render them less soluble or wholly insoluble in water. The necessary reagent for this reaction is preferably included in the dispersion during the azeotroping stage. For instance the surface can be subjected to cross linking.

Instead of, or in addition to modifying the matrix polymer, a polymeric shell can be formed by coacervation. Coacervation techniques are, of course, known for encapsulating a variety of materials and are described in, for instance, GB 1,275,712, 1,475,229 and 1,507,739 and DE 3,545,803.

It is possible, in the invention, to form a coacervate polymeric shell about, for instance, dried enzyme particles by techniques such as those described in the above-mentioned patents. Preferably however a dispersion of enzyme particles in a water immiscible liquid (with the enzyme preferably being distributed throughout a polymeric matrix) is preferably formed by azeotroping as described above, and then an emulsion of droplets of the dispersion is formed in an aqueous medium and the droplets are coated by coacervation with polymeric material while dispersed in the aqueous medium to form a coacervate dispersion. The coacervated particles are preferably stabilised against agglomeration while dispersed in the aqueous medium. This can be achieved by the addition of appropriate stabilising agents but preferably the materials used for forming the coacervate are such as to stabilise the coacervated particles against aggregation. Thus preferably the emulsification of the dispersion into the aqueous coacervating medium is conducted in the absence of any emulsifying agent, adequate initial stability be provided by the coacervating polymer or polymers in the coacervating system and final resistance against agglomeration being provided by the coacervate shell.

Although a coacervate coating can in some instances be formed by precipitation of a single polymer around the emulsified particles it is preferably formed by physical or chemical interaction between the two or more coacervating polymers in the aqueous medium into which the solution of enzyme (and usually matrix polymer) has already been dispersed, for instance to resemble an emulsion. As is known, when two coacervating polymers interact in the presence of an emulsion they tend to form a coating around the individual particles of the emulsion. However any other way of forming a stabilising polymeric coating on the particles by coacervation can be used. For instance a fine particulate coacervate can be formed in an aqueous medium in the absence of the emulsified particles and can then be contacted with the emulsified particles, either by emulsifying the organic solution into the aqueous medium containing the particulate coacervate or by blending that aqueous medium with an aqueous medium containing the emulsified particles. A method of this general type is described in DE-A-3,545,803 (except that it is not essential to react the coacervate coating subsequently with non-ionic melamine formaldehye).

The coacervate-forming polymers that will interact to form a coacervate are generally counterionic. At least one of the polymers may be amphoteric. Particularly preferred polymers are blends of cationic formaldehyde polymer (generally cationic urea formaldehyde) and anionic acrylamide polymer. Suitable materials are described in, for instance, DE-A-3,545,803, GB 2,073,132 and 1,507,739 and U.S. 4,100,103.

For the purposes of the present invention, it is usually sufficient merely to form the dispersion-stabilising coacervate coating around the fluid particles and it is usually unnecessary to react this further by cross linking or condensation with, for instance, further formaldehyde polymer, as is suggested in each of those patents.

Provided the emulsification of the dispersion droplets into the aqueous coacervating medium is conducted with only low amounts of shear and/or in the substantial absence of oil-in-water emulsifier, it is possible to form an emulsion of droplets of the dispersion in the aqueous medium and the eventual coacervated particles will then each consist of the outer coacervated shell, one or more of the enzyme particles, generally being matrix particles having enzyme distributed throughout, and an inner hydrophobic shell between these particles and the outer shell. This inner hydrophobic shell can consist solely of the original water immiscible liquid but preferably the water immiscible liquid that is encapsulated comprises a blend of a relatively volatile liquid and a less volatile hydrophobic material, and the relatively volatile liquid is evaporated from the particles during or after coacervation.

The evaporation can be conducted by distillation at atmospheric or reduced pressure and is generally conducted as an azeotropic distillation. This is generally conducted under reduced pressure. By appropriate selection of the solvent and the pressure at which distillation is conducted it is possible to effect the distillation at low temperatures, e.g., as low as 50°C or even as low as 30°C. It can be convenient to add appropriate volatile liquid and/or less volatile liquid to the azeotroped dispersion from the first stage, before the coacervation. The less volatile hydrophobic material can be either a high boiling oil or can be a solid or semi-solid material, for instance to form a wax layer around the enzyme particles.

The following are examples.

### Example 1

A 25% aqueous solution of ammonium polyacrylate having molecular weight 30,000 is blended with sufficient of a detergent alkaline protease to give a polymer:enzyme dry weight ratio of 19:1. This solution is stirred into a paraffinic oil in the presence of a water-in-oil emulsifier and an amphipathic polymeric stabiliser using sufficient shear to form a stable emulsion in the oil of particles having a size below 3µm and consisting of the aqueous blend of polymer and enzyme.

This emulsion is then be subjected to azeotropic distillation under reduced pressure such that the maximum temperature in the emulsion does not exceed about 50°C, and results in a dispersion in the oil of substantially dry particles having a size below 3µm, often below 1µm, each consisting of a matrix of water soluble polymer, mainly in the free acid form, throughout which the enzyme is uniformly distributed.

This dispersion is stirred gently into a conventional high-surfactant, high-electrolyte, low-water domestic clothes detergent to form a dispersion of the substantially individual polymer-enzyme particles in the detergent. These particles may remain substantially stable during storage but upon dilution with water the polymer will dissolve to expose the enzyme to the other components in the detergent.

### Example 2

The dispersion of enzyme-polymer matrix particles in parrafin oil is obtained as in example 1.

A solution of 168g of 20% aqueous acrylamide/sodium acrylate polymer is dissolved in 600g water and 76g of a 35% urea/formaldehyde resin is dissolved in 100g water and is added over a period of 20 seconds while stirring with a Silverson stirrer, stirring then being continued for a further 30 seconds. 120g of the dispersion in parrafinic oil is then stirred into this solution to form a white emulsion.

This emulsion can then be stirred into a liquid detergent concentrate.

### Example 3

The process of example 2 can be repeated except that a solution in a low boiling hydrocarbon of a waxy hydrocarbon is stirred into the azeotroped dispersion before emulsification in the coacervating polymer solution. The resultant stirred emulsion is then subjected to distillation under reduced pressure at a maximum temperature of 45°C in order to strip off most of the low boiling solvent.

## Claims

1. A liquid composition comprising a substantially stable dispersion in a liquid phase of particles which have a size below 20 µm and which comprise a detergent enzyme and protective polymeric material which is impermeable to liquid detergent concentrates but which releases the enzyme when agitated in aqueous wash liquor and which consists of any one of
(a) a matrix polymer through which the enzyme is distributed, optionally together with an outer shell which is a film of polymeric dispersion stabiliser
(b) an outer shell which is a film of polymeric dispersion stabiliser, and
(c) an outer shell of coacervated polymeric material, optionally together with an inner film of polymeric dispersion stabiliser and in both cases (a) and (b) the particles have been made by a process comprising forming a dispersion of an aqueous phase in a water immiscible liquid in the presence of polymeric dispersion stabiliser wherein the aqueous phase contains the enzyme and azeotroping the dispersion.

2. A composition according to claim 1 in which the protective polymeric material is (a) and in which the matrix polymer is an addition polymer of ethylenically unsaturated ionic monomer optionally with ethylenically unsaturated non-ionic monomer.

3. A composition according to claim 1 or claim 2 in which the protective polymeric material is
(a) a matrix polymer through which the enzyme is distributed together with an outer film of polymeric dispersion stabiliser.

4. A composition according to any preceding claim in which the protective polymeric material is (b).

5. A composition according to claim 3 or claim 4 in which the stabiliser is an amphipathic polymeric stabiliser.

6. A composition according to claim 2 or claim 3 in which the matrix polymer has been formed by including the polymer as a solution or emulsion in the dispersed aqueous phase.

7. A composition according to claim 6 in which the matrix polymer has been chemically modified during or after the azeotroping to render it less permeable to liquid detergent concentrate.

8. A composition according to claim 1 in which the protective polymeric material is (c).

9. A composition according to any of claims 1 to 5 or 8 in which the particles have a size below 3 µm.

10. A composition according to any of claims 1 to 5 or 9 or 10 in which the liquid phase is a liquid detergent.
